## Europäisches Patentamt

## European Patent Office

## Office européen des brevets

(19)

(11) Publication number : **0 436 382 B1**

(12)

# EUROPEAN PATENT SPECIFICATION

(45) Date of publication of patent specification :
06.04.94 Bulletin 94/14

(51) Int. Cl.⁵ : **A61K 6/083**

(21) Application number : 90314288.3

(22) Date of filing : 24.12.90

(54) **Dental material containing anti-slump additive.**

(30) Priority : **03.01.90 US 460356**

(43) Date of publication of application :
10.07.91 Bulletin 91/28

(45) Publication of the grant of the patent :
06.04.94 Bulletin 94/14

(84) Designated Contracting States :
**CH DE ES FR GB IT LI NL SE**

(56) References cited :
EP-A- 0 011 734
EP-A- 0 334 256
US-A- 4 512 743
US-A- 4 782 100
US-A- 4 818 792

(73) Proprietor : **MINNESOTA MINING AND MANUFACTURING COMPANY**
**3M Center, P.O. Box 33427**
**St. Paul, Minnesota 55133-3427 (US)**

(72) Inventor : **Holmes, Brian N., c/o Minnesota Mining and**
**Manufacturing Company, 2501 Hudson Road**
**St. Paul, Minnesota 55144-1000 (US)**

(74) Representative : **Baillie, Iain Cameron et al**
**c/o Ladas & Parry Altheimer Eck 2**
**D-80331 München (DE)**

## Description

TECHNICAL FIELD

This invention relates to dental materials, and to the repair of teeth.

BACKGROUND ART

Plastic-based dental composites have been used for many years in dentistry. Early composites were usually made from a liquid-powder combination of methacrylic acid and poly(methyl methacrylate). Poly(methyl methacrylate) has occasionally been combined with monomers other than methacrylic acid, and in such applications is typically employed at loading levels befitting a composite filler. For example, U.S. Pat. No. 4,308,190 describes dental materials containing 20 to 60 parts by weight polymerizable binder (e.g., diglycidyl methacrylate of Bisphenol A, frequently referred to as "BIS-GMA", and also known as 2,2-bis[4-(2-hydroxy-3-methacryloxypropoxy)phenyl]propane), 20 to 75 parts by weight "crosslinked bead polymer" and 5 to 50 parts by weight fine-particled inorganic filler. The materials are said to be non-tacky and to have a "firm consistency".

U.S. Pat. No. 4,396,476 describes dental compositions containing 0 to 50 weight percent uncrosslinked polymer, 20 to 66 weight percent of a polymerizable monomer capable of dissolving the polymer, 10 to 70 weight percent crosslinked polymer and 0.25 to 27 weight percent crosslinking agent for the polymerizable monomer. Although the '476 patent broadly refers to the use of 0 to 50 weight percent uncrosslinked polymer, it states a preference for 13 to 34 weight percent of uncrosslinked polymer, and exemplifies no uncrosslinked polymer-containing compositions having less than 12 weight percent uncrosslinked polymer. The compositions of the '476 patent are said to have "improved workability".

U.S. Pat. No. 4,552,906 describes dental molding compositions containing, inter alia, 20 to 50 weight percent of a mixture of monofunctional and polyfunctional methacrylic esters, 20 to 60 weight percent essentially non-crosslinked polymer homogeneously filled with microfine inorganic filler and based on (meth)acrylic esters, and 0.5 to 25 weight percent non-crosslinked polymer having a glass transition temperature less than or equal to 0°C and a weight average molecular weight of 1,000 to 500,000.

U.S. Pat. No. 4,648,845 describes primers for use in repairing metal-based restorations, and exemplifies a primer whose solids content is about 70 weight percent of a 200,000 molecular weight copolymer of acrylic acid and ethyl methacrylate, and about 29 weight percent of a 60:40 mixture of BIS-GMA and triethyleneglycol dimethacrylate (the latter methacrylate is frequently referred to as "TEGDMA").

U.S. Pat. No. 4,782,100 describes a prosthetic composition comprising (A) a certain diester monomer or a mixture of said diester monomer and other monomer copolymerizable therewith, (B) an organic polymer soluble in the monomer (A) in an amount of 0.5 to 5 parts by weight per part by weight of said monomer (A) and (C) a radical polymerization initiator.

U.K. Pat. Specification No. 1,478,367 describes orthodontic adhesives containing, inter alia, from 10 to 80 percent of an ester of methacrylic acid with a monoalcohol containing at least one epoxy group, 20 to 90 percent finely divided filler selected from finely divided insoluble organic fillers and finely divided insoluble inorganic fillers, and 10 to 80 percent crosslinking agent. Included in the working examples of the '367 patent are adhesives containing finely-divided cured polymethyl methacrylate, finely-divided poly(ethyl methacrylate), and finely-divided cured 50/50 copolymer of methyl and ethyl methacrylate. The polyacrylate or polymethacrylate polymers represent at least 50 weight percent of the adhesives in the working examples.

T. Tanaka, N. Nakabayashi and E. Masuhara, "Preparation of Clear Thermosetting Resin for Veneered Crown from Several BisMEPP Monomers", Shika Rikosaku Zasshi, 19(47), 168-72 (1978) describes the properties of a mixture of 2,2-bis(p-methacryloxy(ethoxy)$_{2-4}$phenyl)-propane and poly(methyl methacrylate), and the influence of poly(methyl methacrylate) molecular weight on the mixture's transparency. The authors conclude that as the molecular weight of the poly(methyl methacrylate) increases, transparency increases.

Two recent references deal with the properties of the now little-used mixtures of methacrylic acid and poly(methyl methacrylate). T. Hirasawa and K. Nagata, "Studies on the Dental Methacrylic Resins (Part 4), Flow Properties and Curing Times of the Pour Methacrylic Resins", J. Jpn. Soc. Dent. Appar. Mater., 16(35), 73-8 (1975) describes mixtures of poly(methyl methacrylate) and methyl methacrylate, and the effect upon viscosity of the mixture as the particle size and molecular weight of the polymer are varied. The authors conclude that as the molecular weight of the polymer is increased, viscosity of the mixture increases.

T. Hirasawa and T. Nagamitsu, "Studies of the Dental Methacrylic Resins (V)", "Effects of Molecular Weight and Temperature on the Plasticity", Shika Rikosaku Zasshi, 18(42), 110-7 (1977) describes the influence of poly(methyl methacrylate) molecular weight on several properties of a poly(methyl methacrylate)-methyl methacrylate mixture. The authors conclude, inter alia, that "plasticity" is reduced with an increase in molecular weight of the polymer.

## SUMMARY OF THE INVENTION

In general, when the above-references describe combinations of acrylate or methacrylate polymers (e.g., poly(methyl methacrylate)) with a polyfunctional acrylate or methacrylate monomer, they employ relatively large quantities (e.g., 12 weight percent or more) of polymer. The purposes for adding the polymer vary from reference to reference, and sometimes are not stated, but in many cases the polymer is said to reduce tack, or serves as the primary filler. There appears to have been little consideration of the effect of the addition of small amounts of polymer to a filled polyfunctional acrylate or methacrylate resin system. The present invention employs a small amount of acrylic or methacrylic homopolymer or copolymer as an anti-slump additive in a filled polyfunctional acrylate or methacrylate resin system. Very low levels of polymer addition provide significant improvement in slump resistance. The resulting dental materials are easier to place and anatomically shape on or in prepared teeth. The dental materials of the present invention comprise a mixture of:
(a) a major proportion of finely-divided inorganic or finely-divided crosslinked organic filler,
(b) polymerizable polyfunctional acrylate or methacrylate resin, and
(c) acrylate or methacrylate homopolymer or copolymer (for brevity, the "polymer"),
characterized in that the polymer is soluble or swellable in the polymerizable resin, the polymer has at least one $T_g$ greater than 0°C, the resin is present in intermediate proportion, and the polymer is present in minor proportion, and the amount of polymer is sufficient to reduce slump noticeably, but is less than 10 weight percent of the dental material.

The invention also provides a method for improving the slump resistance of a filled dental material.

## DETAILED DESCRIPTION

The dental materials of the present invention are hardenable or hardened compositions suitable for use in the mouth. They can be multiple-part chemical cure or one-part UV- or visible-light-curable compositions. They find particular utility as posterior composites, and also can be used as anterior restoratives, combination-use ("universal") restoratives, cavity liners, orthodontic bracket adhesives, artificial crowns, luting cements and veneers.

The filler (viz., component (a)) is finely divided, and preferably has a maximum particle diameter less than about 50 micrometers and an average particle diameter less than about 10 micrometers. The filler can have a unimodal or polymodal (e.g., bimodal) particle size distribution. The filler can be an inorganic material. It can also be a crosslinked organic material that is insoluble in the polymerizable resin, and is optionally filled with inorganic filler. The filler should in any event be non-toxic and suitable for use in the mouth. The filler can be radiopaque, radiolucent or non-radiopaque.

Examples of suitable inorganic fillers are naturally-occurring or synthetic materials such as quartz, nitrides (e.g., silicon nitride), glass (e.g., fluoroaluminosilicate glass, borosilicate glass and barium glass), hydroxyapatite, ceramic metal oxides (e.g., $CeO_2$, $Sb_2O_5$, $SnO_2$, $ZrO_2$, SrO, BaO, $Al_2O_3$ and $CaCO_3$), low Mohs hardness fillers such as those described in U.S. Pat. No. 4,695,251, and submicron silica particles (e.g., pyrogenic silicas such as the "AEROSIL" Series "OX 50", "130", "150" and "200" silicas sold by Degussa and "CAB-O-SIL M5" silica sold by Cabot Corp.). Examples of suitable organic filler particles include filled or unfilled pulverized polycarbonates, polyepoxides, and the like. Preferred filler particles are quartz, submicron silica, and non-vitreous microparticles of the type described in U.S. Pat. No. 4,503,169.

Preferably the surface of the filler particles is treated with a coupling agent in order to enhance the bond between the filler and the polymerizable resin. The use of such coupling agents is well known in the art. Examples of suitable coupling agents include gamma-methacryloxypropyltrimethoxysilane, gamma-mercaptopropyltriethoxysilane, gamma-aminopropyltrimethoxysilane, and the like.

The filler is present in "major proportion", that is, it is present in numerically larger weight percent than any other component of the dental material of the invention. The desired amount of filler will be referred to as the "loading level" and expressed as a weight percent of filler, excluding its coupling agent, compared to the total weight of the dental material. The loading level will vary depending on the type of filler, the type and amount of other components in the dental material, and the end use of the dental material. Compressive strength and diametral tensile strength generally increase with higher loading levels. In applications such as veneers, cavity liners and orthodontic bracket adhesives the loading level will generally be less than about 80 weight percent, while in applications where high strength or durability are desired (e.g., anterior or posterior restoratives, prostheses, luting cements, artificial crowns, artificial teeth and dentures) the loading level is preferably at least about 60 weight percent and can be as high as about 90 weight percent. For most dental restorative and prosthetic applications a loading level between about 60 and about 85 weight percent is generally preferred.

The polymerizable resin (viz., component (b)) is a hardenable polyfunctional (e.g., difunctional) acrylate or methacrylate resin having sufficient strength, hydrolytic stability and non-toxicity to render it suitable for use in the mouth. Examples of suitable polymerizable resins include those shown in U.S. Pat. Nos. 3,066,112, 3,539,533, 3,629,187, 3,709,866, 3,751,399, 3,766,132, 3,860,556, 4,002,669, 4,259,117, 4,292,029, 4,308,190, 4,327,014, 4,379,695, 4,387,240, 4,404,150, and 4,648,843, and mixtures and derivatives thereof. The polymerizable resin (or at least one of the polymerizable resins, if a mixture of resins is employed), should be a solvent for the polymer, and thus capable of dissolving or swelling the polymer. Mixtures of BIS-GMA and TEGDMA are especially preferred polymerizable resins for use in the present invention.

The polymerizable resin is present in "intermediate proportion", that is, it is present in a numerically lower weight percent than the filler, but in a numerically greater weight percent than the polymer. The amount of polymerizable resin should be adjusted to provide the desired physical and handling properties before and after cure. Such adjustment typically is carried out empirically, based on experience with dental materials of the prior art. A preferred amount of polymerizable resin is at least about 5 weight percent, more preferably about 10 to about 40 weight percent, and most preferobly about 10 to about 30 weight percent based on the total weight of the dental material.

The polymer (viz., component (c)) preferably is derived from, or derivable from, one or more lower (e.g., one to six carbon atom) hydrocarbyl acrylic or methacrylic acid esters. Suitable monomers from which the polymer can be formed include hydrocarbyl esters such as methyl-, ethyl-, n-propyl-, isopropyl-, n-butyl-, sec-butyl-, tert-butyl-, cyclohexyl-, benzyl-, and styryl- acrylate or methacrylate and mixtures thereof. If desired, these monomers can be substituted with non-interfering substituents such as halo, hydroxyl or perfluoroalkyl groups. Polymers of many of these monomers are commercially available, and include "ELVACITE" acrylic resins from E.I. duPont de Nemours and Co. and "PLEXIGLAS" acrylic resins from Rohm and Haas Co. Preferred commercial resins include "ELVACITE" 2008, 2009, 2010, 2021, 2032, 2041, 2042 and 2045.

The polymer should be swellable, or more preferably soluble, in the polymerizable (i.e., unpolymerized) resin. Preferably the polymer is finely divided (e.g., with an average particle diameter less than about 100 micrometers) in order to aid in mixing the polymer with the resin. The polymer preferably has a weight average molecular weight between about 10,000 and about 350,000. More preferably, the weight average molecular weight of the polymer is at least about 25,000. In general, high molecular weight polymers provide more satisfactory handling properties than the same weight of a lower molecular weight polymer.

The polymer has at least one $T_g$ (and preferably all of its $T_g$ values, if there is more than one $T_g$) greater than 0°C, more preferably greater than about 37°C, and most preferably greater than about 60°C. $T_g$ values can be obtained from manufacturer's literature or from standard reference works, or measured by differential scanning calorimetry.

The polymer is present in "minor proportion", that is, it is present in a numerically lower weight percent than either the filler or the polymerizable resin. The amount of the polymer should be sufficient to reduce slump noticeably in a dental material containing the polymer. This amount preferably is evaluated empirically, using a prepared tooth model and a panel of dentists. Dentists are very sensitive to the handling properties of dental materials, and can be relied on to predict with fair accuracy when a material has been rendered noticeably slump-resistant.

Resistance to slump can also be evaluated using a special bench test. A 13.5 g sample of "EXPRESS STD" firmer set silicone impression putty No. 7312 (3M) is cured against a sheet of "SCOTCH" optical lighting film No. 2301 (3M) and then peeled away after the putty has hardened. The cured putty surface has a regular array of parallel V-shaped grooves and a sawtooth cross-section. A 200 mg sample of uncured dental material is placed on a glass slide and embossed with the grooved putty surface. The putty is removed and the glass plate is allowed to stand plate-side-down on a horizontal surface at room temperature for times ranging from 0 seconds to 120 seconds. The dental material is photocured using a 60 second irradiation from a "VISILUX 2" dental curing light (3M). The average surface roughness of the cured dental material is evaluated after each photocure interval using a "SURTRONIC 3" profilometer (Taylor-Hobson) equipped with a standard ("02") pickup No. 112/1502. The profilometer is operated on the 99.9 micrometer range with a 0.8 mm cut off. Measurements are made perpendicular to the direction of the grooves and averaged over readings of three different regions. Slump-prone dental materials will have a measured surface roughness that decreases, often sharply, as the interval before photocuring increases. In severe cases the surface roughness may be relatively low even after a 5 second photocure interval.

This bench test has been found to be very effective for evaluating slump resistance. In general, a slump-resistant dental material will maintain an average surface roughness of at least about 20 micrometers, more preferably at least about 25 micrometers, and most preferably at least about 30 micrometers throughout the 120 second evaluation interval.

At low polymer addition levels, other important physical properties of the dental material such as compressive strength, wear resistance and cure shrinkage are generally unchanged. As the amount of polymer is increased, the incremental or marginal improvement in slump resistance diminishes. Above a certain polymer addition level there is no significant marginal improvement in slump resistance, although other properties of the dental material (e.g., visual opacity) may be adversely affected. Accordingly, the amount of polymer should be less than about 10 weight percent, based on the weight of filler and polymerizable resin in the dental material. The preferred amount of polymer is typically much lower, e.g., less than about 5 weight percent, more preferably less than about 3 weight percent.

The dental materials of the present invention can contain conventional adjuvants such as chemical or photochemical polymerization initiation systems, accelerators, inhibitors, stabilizers, pigments, dyes, viscosity modifiers, extending or reinforcing fillers, surface tension depressants, wetting aids, antioxidants and other ingredients known to those skilled in the art.

The dental materials of the present invention can be mixed like conventional dental materials familiar to those skilled in the art. Ordinarily it is preferred to add the polymer to the stirred polymerizable resin, followed by the stepwise addition of filler increments. Some polymers may be difficult to dissolve in or mix with the polymerizable resin, and in such cases a volatile, removable cosolvent such as methylene chloride, acetone, ethanol, ethyl acetate, methyl ethyl ketone or tetrahydro-furan ("THF") can aid mixing. The cosolvent is subsequently removed from the dental material using vacuum and optional heating. After mixing, the dental materials can be packaged and dispensed using means well known to those skilled in the art, such as multiple-part or one-part packages contained in jars, tubes, amalgamator capsules, pre-dosed tips, syringes, and the like.

The dental materials of the invention are especially desirable posterior composites for use in molars and bicuspid teeth subjected to occlusal wear forces. Preferred amounts of filler, polymerizable resin and polymer in such posterior composites are as follows:

| Ingredient | Preferred weight % | Most preferred weight % |
|---|---|---|
| Filler (without coupling agent) | 60 to 90 | 60 to 85 |
| Polymerizable resin | 40 to 5 | 40 to 12 |
| Polymer | 0.01 to 5 | 0.5 to 3 |

The following illustrative examples are offered to aid understanding of the present invention, and should not be considered as limiting its scope. Unless otherwise indicated, all parts and percentages are by weight.

PREPARATORY

EXAMPLE 1

25.5 Parts silica sol ("LUDOX" LS, E. I. duPont de Nemours & Co.) was acidified by the rapid addition of 0.255 parts concentrated nitric acid. In a separate tank, 12.9 parts ion-exchanged zirconyl acetate (Magnesium Elektron Inc.) were diluted with 20 parts deionized water and the resultant solution acidified with 0.255 parts concentrated nitric acid. The silica sol was pumped into the stirred zirconyl acetate solution and mixed for one hour while filtering the stirred mixture through "CUNO" (Commercial Intertech Corp.) 5 μm (micron) and 1 μm (micron) filters. The stirred, filtered mixture was further filtered through a 1 μm (micron) "HYTREX" (Osmonics, Inc.) filter followed by a 0.22 μm (micron) "BALSTON" (Balston Inc.) filter. The filtrate was poured into trays to a depth of about 25 mm and dried at 65°C. in a forced air oven for about 24 hours. The resulting dried material was removed from the oven and tumbled through a rotary tube furnace (Harper furnace Corporation) preheated to 600°C. 21 Parts of calcined microparticles were obtained. The calcined microparticles were comminuted in a tumbling ball mill until all of the microparticles were less than 10 micrometers in particle diameter. 0.3 Part portions of the milled microparticles were placed in a ceramic sagger and fired in air to 825°C for 1 hour. Firing took place in an electric kiln (Harper Furnace Corporation). The fired microparticles were allowed to cool in air. The cooled microparticles were slurried in hydrolyzed gamma-methacryloxypropyltrimethoxysilane, dried in a forced air oven and screened through a 74 micrometer screen.

A posterior composite paste was prepared by mixing 75.6 parts of the fired, silane-treated microparticles with 24.3 parts of a stock resin solution containing 98.1 percent of a 50:50 mixture of BIS-GMA: TEGDMA: 1% ethyl 4-N,N-dimethylaminobenzoate (EDMAB), 0.6% diphenyliodonium hexafluorophosphate ($\emptyset_2I^+PF_6^-$), 0.2% camphorquinone (CPQ) and 0.1% butylated hydroxytoluene (BHT). Because the treated filler particles contained 11.4% silane, the resulting dental material had a 67% filler particle loading level.

EXAMPLE 1

PEMA Addition

The posterior composite of PREPARATORY EXAMPLE 1 exhibited a tendency to "slump" and thus it was somewhat difficult to form into realistic occlusal anatomy. This shortcoming was remedied by adding "ELVACITE" 2042 200,000-250,000 molecular weight poly(ethyl methacrylate) ("PEMA") to the composite. Composites containing 1% and 1.7% PEMA were prepared. The 1.7% PEMA addition is representative and was performed as follows. 950 Parts of a 14.7% solution of PEMA in TEGDMA were added to 20 parts EDMAB, 4 parts CPQ, 12 parts $\emptyset_2I^+PF_6^-$, 2 parts BHT and 1012 parts of a 90:10 BIS-GMA:TEGDMA mixture. This yielded a stock resin solution containing 7% PEMA and a 50:50 BIS-GMA: TEGDMA ratio. Addition of 4.88 parts of this stock resin solution to 15.12 parts treated filler yielded a dental material containing the same filler loading as in Preparatory Example 1 and 1.7% PEMA.

Similar PEMA additions were made to a variety of commercially available syringe-packaged posterior composites or universal restoratives by dissolving PEMA in methylene chloride at a concentration of 15g/l. The resulting solution was added to the composite or restorative at a one part per hundred parts addition level (unless otherwise noted). The methylene chloride was stripped off under vacuum.

Resistance to slump was evaluated using the profilometer bench test described above. Set out below in TABLE I are the materials evaluated, the interval before photocuring, and the measured surface roughness. Consistently high surface roughness values at each time interval indicate good slump resistance.

## TABLE I

| Material | Interval before photocure (Sec.) | Average surface roughness (Ra, micrometers) |
|---|---|---|
| PREPARATORY EXAMPLE 1 Composite | 0 | 36.2 |
| | 5 | 3.1 |
| | 30 | 1.3 |
| | 60 | 1.4 |
| | 120 | 1.3 |
| PREPARATORY EXAMPLE 1 Composite plus PEMA | 0 | 34.6 |
| | 5 | 35.8 |
| | 30 | 32.6 |
| | 60 | 32.9 |
| | 120 | 33.8 |
| PREPARATORY EXAMPLE 1 Composite plus 1.7% PEMA | 0 | 37.3 |
| | 5 | 34.3 |
| | 30 | 35.3 |
| | 60 | 36.6 |
| | 120 | 30.9 |

| Material | Interval before photocure (Sec.) | Average surface roughness (Ra, micrometers) |
|---|---|---|
| "P-30" Resin Bonded Ceramic[1] | 5 | 17.6 |
| | 30 | 4.3 |
| | 60 | 5.3 |
| | 90 | 4.0 |
| | 120 | 3.3 |
| P-30 Resin Bonded Ceramic with PEMA | 5 | 24.4 |
| | 60 | 24.5 |
| | 120 | 23.0 |
| "P-50" Resin Bonded Ceramic[1] | 5 | 21.0 |
| | 30 | 6.1 |
| | 60 | 5.0 |
| | 90 | 3.3 |
| | 120 | 3.9 |
| P-50 Resin Bonded Ceramic with PEMA | 5 | 34.0 |
| | 60 | 33.3 |
| | 120 | 34.5 |
| "FUL-FIL" composite[2] | 0 | 31.5 |
| | 5 | 30.2 |
| | 30 | 14.8 |
| | 60 | 8.9 |
| | 120 | 5.6 |
| FUL-FIL composite with PEMA | 0 | 29.2 |
| | 5 | 31.1 |
| | 30 | 28.9 |
| | 60 | 29.2 |
| | 120 | 23.1 |
| "PRISMA APH" restorative[3] | 0 | 29.4 |
| | 5 | 28.4 |
| | 30 | 22.3 |
| | 60 | 17.8 |
| | 120 | 11.9 |
| PRISMA APH restorative with PEMA | 0 | 30.1 |
| | 5 | 33.3 |
| | 30 | 28.4 |
| | 60 | 27.0 |
| | 120 | 23.9 |
| "HERCULITE XR" restorative[4] | 5 | 30.3 |
| | 60 | 29.2 |
| | 120 | 32.6 |
| HERCULITE XR restorative with PEMA | 5 | 27.7 |
| | 60 | 27.8 |
| | 120 | 24.3 |

[1] 3M ("Universal" shade).
[2] L. D. Caulk Division of Dentsply International Company ("U-B-62" shade).
[3] L. D. Caulk Division of Dentsply International Company ("U-[LYG]-(B-52)" shade).
[4] Kerr Manufacturing Company.

The data in TABLE I above illustrate the significant improvement in slump resistance obtained by addition of methacrylate homopolymer to the composite of PREPARATORY EXAMPLE 1. Comparable improvements were obtained for the products "P-30" Resin Bonded Ceramic, "P-50" Resin Bonded Ceramic, "FUL-FIL" composite and "PRISMA APH" restorative.

The slump resistance of "HERCULITE XR" restorative was not improved by PEMA addition. As shown in the table, this product has some slump resistance as sold. "HERCULITE XR" restorative was subjected to THF extraction and size exclusion chromatography analysis to determine if it contained PEMA or other acrylic or methacrylic polymer. This analysis is believed to be sufficiently accurate to detect very low levels of polymer addition. None was found in the "HERCULITE XR" restorative sample.

COMPARISON EXAMPLE 1

Using the method of EXAMPLE 1, comparison posterior composites containing varying levels of microfine fumed silica were prepared and evaluated for their slump resistance and visual opacity. The fumed silicas employed were "AEROSIL OX-50" and "AEROSIL R972" silica (Degussa Corp.). Visual opacity values were measured according to the following procedure. Disc-shaped 1 millimeter thick by 20 millimeter diameter samples of composite were cured by exposing them to illumination from a "VISILUX 2" dental curing light (3M) for 60 seconds on each side of the disk at a distance of about 6 millimeters. Light transmission through the thickness of the cured disk was measured using a "MACBETH" transmission densitometer Model TD-903 with the yellow filter in place. The visual opacity for each composite was taken directly from the densitometer reading, and corrected to account for any variation in disc thickness from the nominal 1 mm thickness.

Set out below in TABLE II are the type and amount of fumed silica added to each composite (in the row entitled "Additive:"), the measured slump resistance (in the rows entitled "Average surface roughness, micrometers:") at times between 0 and 120 seconds, and the measured visual opacity for each composite. To aid in comparison, TABLE II also includes data for the composite without any additive, data for the composite together with 1.7% PEMA, and data for the commercially available composites or restoratives "P-50", "HERCULITE XR", "PRISMA APH" and "FUL-FIL".

TABLE II

| Composite or Restorative: | ← ——— Preparatory Example 1 ———→ | | | | | | | "P-50" Composite | "HERCULITE XR" Restorative | "PRISMA APH" Restorative | "FUL-FIL" Composite |
|---|---|---|---|---|---|---|---|---|---|---|---|
| Additive: | —— | 1.2% OX-50 | 2.4% OX-50 | 3.6% OX-50 | 1.2% R972 | 2.4% R972 | 1.7% PEMA | —— | —— | —— | —— |
| Average surface roughness, micrometers: | | | | | | | | | | | |
| at 0 seconds | 36.2 | 31.9 | 31.6 | 34.2 | 35.2 | 23.4 | 37.3 | 31.9 | 28.8 | 29.4 | 31.5 |
| at 5 seconds | 3.1 | 15.1 | 22.1 | 22.3 | 21.8 | 21.0 | 34.3 | 20.6 | 28.1 | 28.4 | 30.2 |
| at 30 seconds | 1.3 | 7.9 | 17.9 | 22.8 | 6.0 | 23.2 | 35.3 | 11.5 | 27.5 | 22.3 | 14.8 |
| at 60 seconds | 1.4 | 1.6 | 14.9 | 17.5 | 2.5 | 25.5 | 36.6 | 7.3 | 30.0 | 17.8 | 8.8 |
| at 120 seconds | 1.3 | 1.4 | 9.8 | 17.1 | 9.5 | 29.6 | 30.9 | 5.2 | 28.4 | 11.9 | 5.6 |
| Visual opacity, optical density per millimeter of thickness: | 0.12 | 0.16 | 0.21 | 0.26 | 0.14 | 0.17 | 0.12 | 0.50 | 0.41 | 0.40 | 0.42 |

EP 0 436 382 B1

TABLE II illustrates that addition of fumed silica to a composite can impart slump resistance, but at the expense of increased visual opacity. The PEMA-containing composite of the invention had both outstanding slump resistance and low visual opacity, and bettered all of the other materials shown in TABLE II.

EXAMPLE 2

Using the method of EXAMPLE 1, methacrylate homopolymers were added to the composite of PREPARATORY EXAMPLE 1. Set out below in TABLE III are the identity of the homopolymer (in the row entitled "Additive:"), the measured slump resistance (in the rows entitled "Average surface roughness, micrometers:") at times between 0 and 120 seconds, and the measured visual opacity for each composite. To aid in comparison, TABLE III includes data for the composite without any homopolymer additive.

TABLE III

|  | ← PREPARATORY EXAMPLE 1 → | | |
| Composite or Restorative: | | | |
| Additive: | 1.71 % PEMA[1] | 1.71 % PEMA[2] | 1.71 % PIBMA[3] |
| Average surface roughness, micrometers: | | | |
| at 0 seconds | 36.2 | 34.8 | 34.3 | 32.0 |
| at 5 seconds | 3.1 | 29.2 | 30.7 | 27.6 |
| at 30 seconds | 1.3 | 31.1 | 31.0 | 8.7 |
| at 60 seconds | 1.4 | 31.2 | 31.5 | 8.6 |
| at 120 seconds | 1.3 | 27.7 | 29.3 | 1.9 |
| Visual opacity, optical density per millimeter of thickness: | 0.12 | 0.11 | 0.12 | 0.13 |

[1] 25,000 M.W. poly(ethyl methacrylate), ("ELVACITE" 2043, E.I. duPont de Nemours & Co.).

[2] 350,000 M.W. poly(ethyl methacrylate), (Aldrich Chemicals Co.).

[3] 150,000 M.W. poly(isobutyl methacrylate), ("ELVACITE" 2045, E.I. duPont de Nemours & Co.).

The words "AEROSIL", "BALSTON", "CAB-O-SIL", "CUNO", "ELVACITE", "EXPRESS", "FUL-FIL", " HERCULITE", "HYTREX", "LUDOX", "MACBETH", "P-30", "P-50", "PLEXIGLAS", "PRISMA", "SCOTCH", "SURTRONIC" AND "VISILUX" are trademarks.

## Claims

**Claims for the following Contracting States : CH, DE, FR, GB, IT, LI, NL, SE**

1. Dental materials comprising a mixture of:
   (a) a major proportion of finely-divided inorganic or finely-divided crosslinked organic filler,
   (b) polymerizable polyfunctional acrylate or methacrylate resin, and
   (c) acrylate or methacrylate homopolymer or copolymer,
   characterized in that the homopolymer or copolymer is soluble or swellable in the polymerizable resin, the homopolymer or copolymer has at least one $T_g$ greater than 0°C, the resin is present in intermediate proportion, the homopolymer or copolymer is present in minor proportion, and the amount of homopolymer or copolymer is sufficient to reduce slump noticeably but is less than 10 weight percent of the dental material.

2. Dental materials according to claim 1, characterized in that the homopolymer or copolymer comprises a polymer of methyl-, ethyl-, n-propyl-, isopropyl-, n-butyl-, sec-butyl-, tert-butyl-, cyclohexyl-, benzyl- or styryl- acrylate or methacrylate.

3. Dental materials according to any preceding claim, characterized in that the homopolymer or copolymer comprises methacrylate homopolymer having a $T_g$ greater than 37°C.

4. Dental materials according to any preceding claim, characterized in that the homopolymer comprises poly(ethyl methacrylate).

5. Dental materials according to any preceding claim, characterized in that the homopolymer or copolymer has a weight average molecular weight of 10,000 to 350,000.

6. Dental materials according to any preceding claim, further characterized in that a room temperature sample of the material, if embossed with a grating having sufficient roughness to impart to the sample an average surface roughness of 30 to 40 micrometers, will maintain an average surface roughness of at least 20 micrometers when horizontally disposed at room temperature for two minutes.

7. Dental materials according to claim 6, characterized in that the dental material will maintain an average surface roughness of at least 30 micrometers.

8. Dental materials according to any preceding claim, characterized by containing 60 to 90 weight percent filler, 40 to 5 weight percent polymerizable resin, and 0.1 to 5 weight percent homopolymer or copolymer.

9. Dental materials according to claim 8, characterized by containing 60 to 85 weight percent filler, 40 to 12 weight percent polymerizable resin, and 0.5 to 3 weight percent homopolymer or copolymer.

10. A method for improving the slump resistance of a filled dental material, characterized by the step of adding thereto acrylate or methacrylate homopolymer or copolymer having at least one $T_g$ greater than 0°C, the amount of homopolymer or copolymer being sufficient to reduce slump noticeably but being less than 10 weight percent of the dental material.

**Claims for the following Contracting State : ES**

1. A method for the production of dental arterials comprising a mixture of:
   (a) a major proportion of finely-divided inorganic or finely-divided crosslinked organic filler,
   (b) polymerizable polyfunctional acrylate or methacrylate resin, and
   (c) acrylate or methacrylate homopolymer or copolymer,
   characterized in that the homopolymer or copolymer is soluble or swellable in the polymerizable resin, the homopolymer or copolymer has at least one $T_g$ greater than 0°C, the resin is present in intermediate proportion, the homopolymer or copolymer is present in minor proportion, and the amount of homopolymer or copolymer is sufficient to reduce slump noticeably but is less than 10 weight percent of the dental material.

2. The method according to claim 1, characterized in that the homopolymer or copolymer comprises a polymer of methyl-, ethyl-, n-propyl-, isopropyl-, n-butyl-, sec-butyl-, tert-butyl-, cyclohexyl-, benzyl- or styryl-acrylate or methacrylate.

3. The method according to any preceding claim, characterized in that the homopolymer or copolymer comprises methacrylate homopolymer having a $T_g$ greater than 37°C.

4. The method according to any preceding claim, characterized in that the homopolymer comprises poly(ethyl methacrylate).

5. The method according to any preceding claim, characterized in that the homopolymer or copolymer has a weight average molecular weight of 10,000 to 350,000.

6. The method according to any preceding claim, further characterized in that a room temperature sample of the material, if embossed with a grating having sufficient roughness to impart to the sample an average surface roughness of 30 to 40 micrometers, will maintain an average surface roughness of at least 20 micrometers when horizontally disposed at room temperature for two minutes.

7. The method according to claim 6, characterized in that the dental material will maintain an average surface roughness of at least 30 micrometers.

8. The method according to any preceding claim, characterized by containing 60 to 90 weight percent filler, 40 to 5 weight percent polymerizable resin, and 0.1 to 5 weight percent homopolymer or copolymer.

9. The method according to claim 8, characterized by containing 60 to 85 weight percent filler, 40 to 12 weight percent polymerizable resin, and 0.5 to 3 weight percent homopolymer or copolymer.

10. A method for improving the slump resistance of a filled dental material, characterized by the step of adding thereto acrylate or methacrylate homopolymer or copolymer having at least one $T_g$ greater than 0°C, the amount of homopolymer or copolymer being sufficient to reduce slump noticeably but being less than 10 weight percent of the dental material.

**Patentansprüche**

**Patentansprüche für folgende Vertragsstaaten : CH, DE, FR, GB, IT, NL, SE**

1. Dentalwerkstoffe, die wenigstens teilweise aus einem Gemisch von
   (a) einer größeren Teilmenge aus einem feinverteilten anorganischen oder feinverteilten vernetzten organischen Füllstoff,
   (b) einem polymerisierbaren mehrfunktionellen Acrylat- oder Methacrylatharz und
   (c) einem Acrylat- oder Methacrylathomopolymer oder -copolymer
   bestehen, dadurch gekennzeichnet, daß das Homopolymer oder Copolymer in dem polymerisierbaren Harz löslich oder quellfähig ist, daß das Homopolymer oder Copolymer mindestens eine über 0°C liegende Einfriertemperatur hat, daß das Harz in einer mittleren Teilmenge vorhanden ist, daß das Homopolymer oder Copolymer in einer kleineren Teilmenge vorhanden ist und daß die Menge des Homopolymers oder Copolymers zum merklichen Herabsetzen des Setzens genügt, oder weniger als 10 Gew.-% des Dentalwerkstoffes beträgt.

2. Dentalwerkstoffe nach Anspruch 1, dadurch gekennzeichnet, daß das Homopolymer oder Copolymer wenigstens teilweise aus einem Polymer von Methyl-, Ethyl-, n-Propyl-, Isopropyl-, n-Butyl-, sek. Butyl-, tert. Butyl-, Cyclohexyl-, Benzyl- oder Styrylacrylat oder -methacrylat besteht.

3. Dentalwerkstoffe nach einem der vorhergehenden Ansprüche, dadurch gekennzeichnet, daß das Homopolymer oder Copolymer wenigstens teilweise aus einem Methacrylathomopolymer mit einer Einfriertemperatur über 37°C besteht.

4. Dentalwerkstoffe nach einem der vorhergehenden Ansprüche, dadurch gekennzeichnet, daß das Homopolymer wenigstens teilweise aus Poly(ethylmethacrylat) besteht.

5. Dentalwerkstoffe nach einem der vorhergehenden Ansprüche, dadurch gekennzeichnet, daß das Homopolymer oder Copolymer ein gewichtsdurchschnittliches Molekulargewicht von 10 000 bis 350 000 hat.

6. Dentalwerkstoffe nach einem der vorhergehenden Ansprüche, dadurch gekennzeichnet, daß bei einem auf Zimmertemperatur befindlichen Probekörpers des Werkstoffes nach seinem Prägen mit einem Gitter, dessen Rauhtiefe genügt, um dem Probekörper eine durchschnittliche Oberflächenrauhtiefe von 30 bis 40 Mikrometern zu verleihen, eine durchschnittliche Rauhtiefe von mindestens 20 Mikrometern aufrechterhalten wird, wenn der Probekörper zwei Minuten bei Zimmertemperatur horizontal angeordnet wird.

7. Dentalwerkstoffe nach Anspruch 6, dadurch gekennzeichnet, daß in dem Dentalwerkstoff eine durchschnittliche Rauhtiefe von mindestens 30 Mikrometern aufrechterhalten wird.

8. Dentalwerkstoffe nach einem der vorhergehenden Ansprüche, gekennzeichnet durch einen Gehalt von 60 bis 90 Gew.-% Füllstoff, 40 bis 5 Gew.-% polymerisierbarem Harz und 0.1 bis 5 Gew.-% Homopolymer oder Copolymer.

9. Dentalwerkstoffe nach Anspruch 8, gekennzeichnet durch einen Gehalt von 60 bis 85 Gew.-% Füllstoff, 40 bis 12 Gew.-% polymerisierbarem Harz und 0.5 bis 3 Gew.-% Homopolymer oder Copolymer.

10. Verfahren zum Verbessern der Setzfestigkeit eines Füllstoffhaltigen Dentalwerkstoffes, dadurch gekennzeichnet, daß ihm ein Acrylat- oder Methacrylathomopolymer oder -copolymer mit mindestens einer Einfriertemperatur über 0°C in einer Menge zugesetzt wird, die zum merklichen Herabsetzen des Setzens genügt, aber weniger als 10 Gew.-% des Dentalwerkstoffes beträgt.

**Patentansprüche für folgenden Vertragsstaat : ES**

1. Verfahren zum Herstellen von Dentalwerkstoffen, die wenigstens teilweise aus einem Gemisch von
    (a) einer größeren Teilmenge aus einem feinverteilten anorganischen oder feinverteilten vernetzten organischen Füllstoff,
    (b) einem polymerisierbaren mehrfunktionellen Acrylat- oder Methacrylatharz und
    (c) einem Acrylat- oder Methacrylathomopolymer oder -copolymer
bestehen, dadurch gekennzeichnet, daß das Homopolymer oder Copolymer in dem polymerisierbaren Harz löslich oder quellfähig ist, daß das Homopolymer oder Copolymer mindestens eine über 0°C liegende Einfriertemperatur hat, daß das Harz in einer mittleren Teilmenge vorhanden ist, daß das Homopolymer oder Copolymer in einer kleineren Teilmenge vorhanden ist und daß die Menge des Homopolymers oder Copolymers zum merklichen Herabsetzen des Setzens genügt, oder weniger als 10 Gew.-% des Dentalwerkstoffes beträgt.

2. Verfahren nach Anspruch 1, dadurch gekennzeichnet, daß das Homopolymer oder Copolymer wenigstens teilweise aus einem Polymer von Methyl-, Ethyl-, n-Propyl-, Isopropyl-, n-Butyl-, sek. Butyl-, tert. Butyl-, Cyclohexyl-, Benzyl- oder Styrylacrylat oder -methacrylat besteht.

3. Verfahren nach einem der vorhergehenden Ansprüche, dadurch gekennzeichnet, daß das Homopolymer oder Copolymer wenigstens teilweise aus einem Methacrylathomopolymer mit einer Einfriertemperatur über 37°C besteht.

4. Verfahren nach einem der vorhergehenden Ansprüche, dadurch gekennzeichnet, daß das Homopolymer wenigstens teilweise aus Poly(ethylmethacrylat) besteht.

5. Verfahren nach einem der vorhergehenden Ansprüche, dadurch gekennzeichnet, daß das Homopolymer oder Copolymer ein gewichtsdurchschnittliches Molekulargewicht von 10 000 bis 350 000 hat.

6. Verfahren nach einem der vorhergehenden Ansprüche, dadurch gekennzeichnet, daß bei einem auf Zimmertemperatur befindlichen Probekörpers des Werkstoffes nach seinem Prägen mit einem Gitter, dessen Rauhtiefe genügt, um dem Probekörper eine durchschnittliche Oberflächenrauhtiefe von 30 bis 40 Mikrometern zu verleihen, eine durchschnittliche Rauhtiefe von mindestens 20 Mikrometern aufrechterhalten wird, wenn der Probekörper zwei Minuten bei Zimmertemperatur horizontal angeordnet wird.

7. Verfahren nach Anspruch 6, dadurch gekennzeichnet, daß in dem Dentalwerkstoff eine durchschnittliche Rauhtiefe von mindestens 30 Mikrometern aufrechterhalten wird.

**8.** Verfahren nach einem der vorhergehenden Ansprüche, gekennzeichnet durch einen Gehalt von 60 bis 90 Gew.-% Füllstoff, 40 bis 5 Gew.-% polymerisierbarem Harz und 0.1 bis 5 Gew.-% Homopolymer oder Copolymer.

**9.** Verfahren nach Anspruch 8, gekennzeichnet durch einen Gehalt von 60 bis 85 Gew.-% Füllstoff, 40 bis 12 Gew.-% polymerisierbarem Harz und 0.5 bis 3 Gew.-% Homopolymer oder Copolymer.

**10.** Verfahren zum Verbessern der Setzfestigkeit eines füllstoffhaltigen Dentalwerkstoffes, dadurch gekennzeichnet, daß ihm ein Acrylat- oder Methacrylathomopolymer oder -copolymer mit mindestens einer Einfriertemperatur über 0°C in einer Menge zugesetzt wird, die zum merklichen Herabsetzen des Setzens genügt, aber weniger als 10 Gew.-% des Dentalwerkstoffes beträgt.

## Revendications

**Revendications pour les Etats contractants suivants : CH, DE, FR, GB, IT, LI, NL, SE**

**1.** Matériaux dentaires comprenant un mélange de :
(a) une proportion importante d'une charge minérale finement divisée ou d'une charge organique réticulée finement divisée,
(b) une résine d'acrylate ou de méthacrylate polyfonctionnelle et polymérisable, et
(c) un homopolymère ou copolymère d'acrylate ou de méthacrylate,
caractérisés en ce que l'homopolymère ou copolymère est soluble ou gonflable dans la résine polymérisable, l'homopolymère ou copolymère a au moins une $T_g$ supérieure à 0° C, la résine est présente selon une proportion intermédiaire, l'homopolymère ou copolymère est présent selon une proportion mineure, et la quantité d'homopolymère ou copolymère est suffisante pour réduire notablement l'affaissement mais est inférieure à 10 % en poids du matériau dentaire.

**2.** Matériaux dentaires suivant la revendication 1, caractérisés en ce que l'homopolymère ou copolymère comprend un polymère d'acrylate ou de méthacrylate de méthyle, éthyle, n-propyle, isopropyle, n-butyle, s.-butyle, t.-butyle, cyclohexyle ou styryle.

**3.** Matériaux dentaires suivant l'une quelconque des revendications précédentes, caractérisés en ce que l'homopolymère ou copolymère comprend un homopolymère de méthacrylate ayant une $T_g$ supérieure à 37° C.

**4.** Matériaux dentaires suivant l'une quelconque des revendications précédentes, caractérisés en ce que l'homopolymère comprend le poly(méthacrylate d'éthyle).

**5.** Matériaux dentaires suivant l'une quelconque des revendications précédentes, caractérisés en ce que l'homopolymère ou copolymère a un poids moléculaire moyen en poids de 10 000 à 350 000.

**6.** Matériaux dentaires suivant l'une quelconque des revendications précédentes, caractérisés en ce que, en outre, un échantillon du matériau à la température ambiante, s'il a été bosselé avec un réseau (en anglais : "grating") ayant une rugosité suffisante pour fournir audit échantillon une rugosité de surface de 30 à 40 μm, conservera une rugosité moyenne de surface d'au moins 20 μm s'il est disposé horizontalement à la température ambiante pendant 2 minutes.

**7.** Matériaux dentaires suivant la revendication 6, caractérisés en ce que le matériau dentaire conservera une rugosité moyenne de surface d'au moins 30 μm.

**8.** Matériaux dentaires suivant l'une quelconque des revendications précédentes, caractérisés en ce qu'ils contiennent 60 à 90 % en poids de charge, 40 à 5 % en poids de résine polymérisable, et 0,1 à 5 % en poids d'homopolymère ou copolymère.

**9.** Matériaux dentaires suivant l'une quelconque des revendications précédentes, caractérisés en ce qu'ils contiennent 60 à 85 % en poids de charge, 40 à 12 % en poids de résine polymérisable, et 0,5 à 3 % en poids d'homopolymère ou copolymère.

10. Procédé pour améliorer la résistance à l'affaissement d'un matériau dentaire de garniture, caractérisé par l'étape d'addition au dit matériau d'un homopolymère ou copolymère d'acrylate ou de méthacrylate ayant au moins une $T_g$ supérieure à 0° C, la quantité d'homopolymère ou copolymère étant suffisante pour réduire notablement l'affaissement mais étant inférieure à 10 % en poids du matériau dentaire.

**Revendications pour l'Etat contractant suivant : ES**

1. Procédé pour la production de matériaux dentaires comprenant un mélange de :
   (a) une proportion importante d'une charge minérale finement divisée ou d'une charge organique réticulée finement divisée,
   (b) une résine d'acrylate ou de méthacrylate polyfonctionnelle et polymérisable, et
   (c) un homopolymère ou copolymère d'acrylate ou de méthacrylate,
   caractérisé en ce que l'homopolymère ou copolymère est soluble ou gonflable dans la résine polymérisable, l'homopolymère ou copolymère a au moins une $T_g$ supérieure à 0° C, la résine est présente selon une proportion intermédiaire, l'homopolymère ou copolymère est présent selon une proportion mineure, et la quantité d'homopolymère ou copolymère est suffisante pour réduire notablement l'affaissement mais est inférieure à 10 % en poids du matériau dentaire.

2. Procédé suivant la revendication 1, caractérisé en ce que l'homopolymère ou copolymère comprend un polymère d'acrylate ou de méthacrylate de méthyle, éthyle, n-propyle, isopropyle, n-butyle, s.-butyle, t.-butyle, cyclohexyle ou styryle.

3. Procédé suivant l'une quelconque des revendications précédentes, caractérisé en ce que l'homopolymère ou copolymère comprend un homopolymère de méthacrylate ayant une $T_g$ supérieure à 37° C.

4. Procédé suivant l'une quelconque des revendications précédentes, caractérisé en ce que l'homopolymère comprend le poly(méthacrylate d'éthyle).

5. Procédé suivant l'une quelconque des revendications précédentes, caractérisé en ce que l'homopolymère ou copolymère a un poids moléculaire moyen en poids de 10 000 à 350 000.

6. Procédé suivant l'une quelconque des revendications précédentes, caractérisé en ce que, en outre, un échantillon du matériau à la température ambiante, s'il a été bosselé avec un réseau (en anglais : "grating") ayant une rugosité suffisante pour fournir audit échantillon une rugosité de surface de 30 à 40 μm, conservera une rugosité moyenne de surface d'au moins 20 μm s'il est disposé horizontalement à la température ambiante pendant 2 minutes.

7. Procédé suivant la revendication 6, caractérisé en ce que le matériau dentaire conservera une rugosité moyenne de surface d'au moins 30 μm.

8. Procédé suivant l'une quelconque des revendications précédentes, caractérisé en ce que les matériaux dentaires contiennent 60 à 90 % en poids de charge, 40 à 5 % en poids de résine polymérisable, et 0,1 à 5 % en poids d'homopolymère ou copolymère.

9. Procédé suivant l'une quelconque des revendications précédentes, caractérisé en ce que les matériaux dentaires contiennent 60 à 85 % en poids de charge, 40 à 12 % en poids de résine polymérisable, et 0,5 à 3 % en poids d'homopolymère ou copolymère.

10. Procédé pour améliorer la résistance à l'affaissement d'un matériau dentaire de garniture, caractérisé par l'étape d'addition au dit matériau d'un homopolymère ou copolymère d'acrylate ou de méthacrylate ayant au moins une $T_g$ supérieure à 0° C, la quantité d'homopolymère ou copolymère étant suffisante pour réduire notablement l'affaissement mais étant inférieure à 10 % en poids du matériau dentaire.